# EUROPEAN PATENT SPECIFICATION

(11) **EP 0 284 132 B1**
(45) Date of publication and mention of the grant of the patent: **12.01.1994**
(21) Application number: 88200430.2
(22) Date of filing: 08.03.1988
(51) Int. Cl.: C11D 3/39

(54) **Quaternary ammonium or phosphonium peroxy carbonic acid precursors and their use in detergent bleach compositions**
Quartäre Ammonium- oder Phosphonium-Peroxycarbonsäure-Prekursoren und ihre Verwendung in Detergensbleichmittelzusammensetzungen
Précurseurs d'acide peroxycarbonique d'ammonium quaternaire ou de phosphonium et leur utilisation dans des compositions de détergents de blanchiment

(30) Priority: 17.03.1987 US 27278
(43) Date of publication of application: 28.09.1988
(73) Proprietor: UNILEVER N.V., 3013 AL Rotterdam (NL); UNILEVER PLC, London EC4P 4BQ (GB)
(72) Inventor: Humphreys, Robert William Riley, Oradell New Jersey (US); Madison, Stephen Alan, Valley Cottage New York 10989 (US)
(74) Representative: Kan, Jacob Hendrik, Dr.

(56) References cited:
- EP-A- 0 185 522
- US-A- 4 260 529

## Description

### BACKGROUND OF THE INVENTION

### 1. Field of the Invention

The invention relates to novel bleach precursors, peracids generated therefrom and use of these materials in detergent compositions.

### 2. The Prior Art

It is well known that active oxygen-releasing compounds are effective bleaching agents. These compounds are frequently incorporated in detergent compositions for stain and soil removal. Unlike the traditional sodium hypochlorite bleaches, oxygen-releasing compounds are less aggressive and thus more compatible with detergent compositions. They have, however, an important limitation: the activity of these compounds is extremely temperature-dependent. Thus, oxygen-releasing bleaches are essentially only practical when the bleaching solution is heated above 60°C. At a temperature of just 60°C, extremely high amounts of the active oxygen-releasing compounds must be added to the system to achieve any bleach effect. Although this would indicate the desirability of high temperature operation, high temperatures are both economically and practically disadvantageous.

At bleach solution temperatures below 60°C, the active oxygen-releasing compounds are rendered much less effective regardless of their level in the system. With respect to bleaching of laundry in automatic household washing machines, it must be noted that these machines are normally operated at wash-water temperatures below 60°C. Consequently, there has developed a need for substances which promote release of active oxygen at temperatures below 60°C. These substances are generally referred to in the art as bleach precursors, although they have also been called promoters and activators. Normally, bleach precursors are used in conjunction with persalts capable of releasing hydrogen peroxide in aqueous solution, perborate being the most widely used persalt.

Typically, the precursor is a reactive compound such as a carboxylic acid ester that in alkaline solution containing a source of hydrogen peroxide, e.g. a persalt, will generate the corresponding peroxy acid. The reaction involves nucleophilic substitution on to the precursor by hydroperoxy anions (HOO⁻) and is facilitated by precursors having good leaving groups. Often the reaction is referred to as perhydrolysis.

Early patents in the area of precursor chemistry include US Patent 3,256,198 and US Patent 3,272,750, each of which suggests the use of organic carbonate esters as bleach aids. British Patent 836,988 and British Patent 864,798 were forerunners disclosing the use of aliphatic carboxylic acid esters as adjuncts for accelerating the bleaching of persalts such as sodium perborate or percarbonate.

US Patent 4,283,301 discloses a peroxygen bleach and a precursor of the general formula: 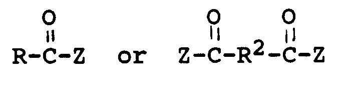
wherein R is an alkyl chain containing from 5 to 13 carbon atoms, R² is an alkyl chain containing from 4 to 24 carbon atoms and each Z is a leaving group as defined therein.

US Patent 4,412,934 reports compositions incorporating bleach precursors of the general formula: 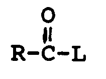
wherein R is an alkyl group containing from 5 to 18 carbon atoms and L is a leaving group.

Similar disclosures are found in US Patent 4,486,327, EP 0 098 129, EP 0 106 584, EP 0 106 634, EP 0 120 591, EP 0 163 331, EP 0 166 571, EP 0 185 522, EP 0 170 386, EP 0 153 222, EP 0 150 223 and EP 0 202 698. Among the preferred leaving groups are those having solubilizing functionality including sulphonic, sulphuric, carboxylate and quaternary ammonium salt groups.

A typical precursor within the concept of the aforedescribed patents is sodium n-nonanoyloxybenzene sulphonate presently commercialized as a component of a branded detergent. This sulphonate, in combination with sodium perborate, effectively releases peroxygen fragments upon perhydrolysis, as well as sodium 4-sulphophenol. Once released, the p-sulphophenol fragment unfortunately provides no additional fabric washing benefit.

Esters such as sodium n-nonanoyloxybenzene sulphonate are reported to require greater than stoichiometric amounts of alkaline hydrogen peroxide. For example, US Patent 4,536,314 discloses hydrogen peroxide/activator ratios ranging from greater than 1.5:1 to 10:1. High peroxide ratios are necessary with these activators to ensure high rates of peracid formation and to account for the unavoidable depletion of peroxide by natural soils. These high ratios are economically wasteful.

US Patent 3,686,127 recognizes the shortcomings of precursors, the leaving groups of which provide no additional fabric washing benefit. Therefore, the patent suggests use of alkylated sulphophenol carboxylic esters which release leaving groups that provide detergent and emulsifying properties. However, with this modification to the leaving group structure, the yield of peracid falls to essentially non-useful levels. For instance, sodium 2-acetoxy-5-hexylbenzene-sulphonate yields 43% peracid after 5 minutes but the unsubstituted derivative yields 80% peracid. Presumably, unfavourable steric or electrostatic interactions arising from the alkyl substituents retard the rate of perhydrolysis.

US Patent 4,397,757 reports that having quaternary ammonium groups on the precursor is advantageous because it allows precursor and intermediate species to substantively attach on to surfaces undergoing bleaching, e.g. fabric surfaces. Substantivity was said to lead to enhanced stain removal, particularly at low temperature. A drawback of this technology is the expense in preparing the precursors; the synthesis involves several steps and requires excess reagent. Starting materials are also not readily available.

While the aforementioned precursors have all been reported effective at stain removal, there is still need of more efficient systems. Stain removal efficiency may be improved either by a precursor that generates equivalent bleach at a lower precursor molar level or operates at lower levels of hydrogen peroxide source. Not only do lower levels of peroxide source or precursor provide better economics, they also permit increased flexibility in detergent formulation.

Consequently, it is an object of the present invention to provide a detergent bleach composition with a precursor that permits bleaching over a wide temperature range including that of under 60°C.

It is another object of the present invention to provide certain novel bleach precursors which have hitherto not been described in the art.

A further object of the present invention is to provide a precursor having a group capable of imparting additional benefits to treated substances including that of detergency and/or fabric softening while still achieving high peracid-generating levels.

Another object of the present invention is to provide a precursor that can be economically synthesized from readily available starting materials and in a minimum number of synthetic steps.

A final object of the present invention is to provide novel peroxy acids generated from the bleach precursors by perhydrolysis with hydrogen peroxide or persalts.

### SUMMARY OF THE INVENTION

A bleach precursor compound is provided having the formula: 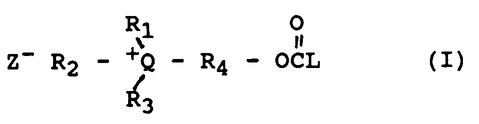
wherein:
R₁, R₂ and R₃ are each a radical selected from the group consisting of alkyl, alkenyl, alkynyl, cycloalkyl, cycloalkenyl, alkaryl, aryl, phenyl, hydroxyalkyl, polyoxyalkylenyl and R₄OCOL;
or two or more of R₁, R₂, and R₃ together form an alkyl-substituted or unsubstituted, nitrogen-containing heterocyclic ring system;
or at least one of R₁, R₂, and R₃ is attached to R₄ to form an alkyl-substituted or unsubstituted, nitrogen-containing hetercyclic ring system;
R₄ is selected from a bridging group consisting of alkylene, cycloalkylene, alkylenephenylene, phenylene, arylene, and polyalkoxylene and wherein the bridging group can be unsubstituted or substituted with C₁-C₂₀ alkyl, alkenyl, benzyl, phenyl and aryl radicals;
Z⁻ is a monovalent or multivalent anion leading to charge neutrality when combined with Q⁺ in the appropriate ratio and wherein Z⁻ is sufficiently oxidatively stable not to interfere significantly with bleaching by a peroxy carbonic acid;
Q is nitrogen or phosphorus; and
L is a leaving group selected from the group consisting of: 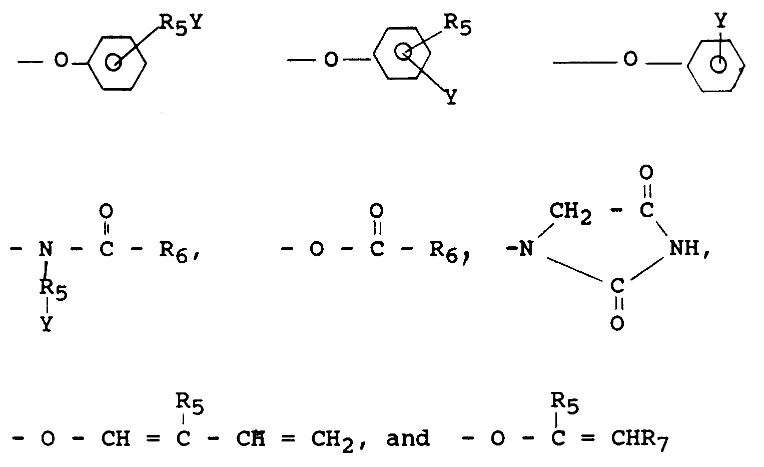
wherein:
R₅ is a C₁-C₁₂ alkyl or alkylene group, R₆ is a C₁-C₁₂ alkyl group, R₇ is H or R₅, and Y is H or a water-solubilizing group, selected from -SO⁻₃M⁺, -COO⁻M⁺, -SO⁻₄M⁺, -N⁺(R₅)₃X⁻, -NO₂, -OH, and O←N(R₅)₂ and mixtures thereof;
wherein M⁺ is a hydrogen, alkali metal, ammonium or alkyl or hydroxyalkyl-substituted ammonium cation. X⁻ is a halide, hydroxide, phosphate, sulphate, methyl sulphate or acetate anion.

A peroxygen acid is also provided having the formula: 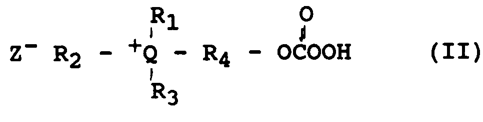
Furthermore, a detergent bleaching composition is provided comprising:
i) from 1 to 60% of a peroxygen compound capable of yielding hydrogen peroxide in an aqueous solution;
ii) from 0.1 to 40% of the bleach precursor of formula I described hereinabove;
iii) from 0 to 50% of a surfactant; and
iv) from 0 to 70% of a detergent builder.

### DETAILED DESCRIPTION OF THE INVENTION

There have now been discovered a novel group of compounds having the formula: 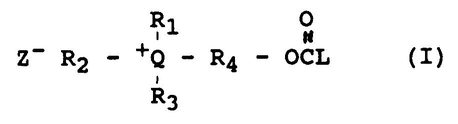
which meet many of the objectives outlined. Peroxy carbonic acid precursors of the formula I have been found to generate peroxy carbonic acids that are superior bleaching agents, giving substantially higher levels of stain removal for a given level of persalt than observed with known precursors.

A most important component of precursors compound (I) is the leaving group (L). Leaving groups of the appropriate structure facilitate reaction of the bleach precursor with hydrogen peroxide in basic aqueous solution to generate a peroxy carbonic acid bleach as follows: 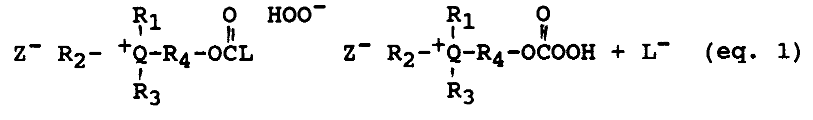
Leaving groups effective for the present invention will induce rapid formation of the peroxy carbonic acid in the presence of a peroxygen source under practical conditions, e.g. in detergent solution during laundering of clothes. Generally, L must be of an electron-attracting structure which promotes successful nucleophilic attack by the perhydroxide anion. Leaving groups which exhibit such properties are those in which the conjugate acid has a pKₐ in the range of from about 6 to about 13, preferably from about 7 to about 11, most preferably from about 8 to about 11.

Many and diverse leaving group structures have been described in the patent literature and are useful for this invention. For example, US Patent 4,412,934, US Patent 4,483,778, European Patent Application 170,386 and European Patent Application 166,571 provide examples of desirable leaving groups and are herein incorporated by way of reference.

The compounds of the invention have leaving structure L selected from the group consisting of: 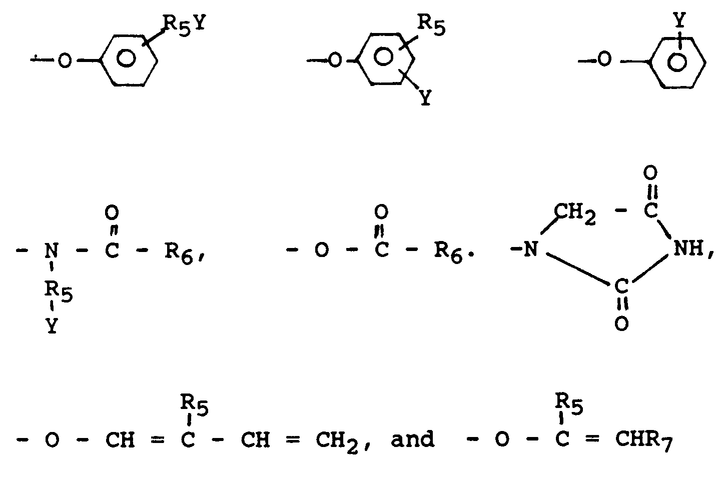
wherein:
R₅ is a C₁-C₁₂ alkyl or alkylene group, R₆ is a C₁-C₁₂ alkyl group, R₇ is H or R₅, and Y is H or a water-solubilizing group, selected from -SO⁻₃M⁺, -COO⁻M⁺, -SO⁻₄M⁺, -N⁺(R₅)₃X⁻,-NO₂,-OH, and O←N(R₅)₂ and mixtures thereof;
wherein M⁺ is a hydrogen, alkali metal, ammonium or alkyl or hydroxyalkyl-substituted ammonium cation. X⁻ is a halide, hydroxide, phosphate, sulphate, methyl sulphate or acetate anion.

Most preferred of the leaving groups is the phenol sulphonate type. Especially preferred is the 4-sulphophenol group. Sodium, potassium and ammonium cations are the preferred counter-ions to the sulphophenol structures.

Although phosphonium groups where Q is phosphorus are within the scope of this invention, for economic reasons it is most preferred that Q be nitrogen. Furthermore, the precursor and respective peracid derivative compounds should preferably contain a quaternary ammonium cation surrounded by R₁, R₂ and R₃, each the same or different and having C₁-C₂₀ atom radicals selected from the group consisting of alkyl, alkylaryl, benzyl, hydroxyalkyl, heterocyclic rings containing the quaternary nitrogen groups where R₁ and R₄ or R₁ and R₂ are joined together, and mixtures of groups thereof.

In particular, it is desirable that R₁ be a short-chain C₁-C₄ alkyl radical, preferably methyl, while R₂ and R₃ be a longer chain C₇-C₂₀ alkyl or alkylaryl, such as a stearyl, lauryl, or benzyl group. With regard to the R₄ bridge between the quaternary nitrogen and carbonate groups, it is desirable that R₄ be a bridging group selected from C₂-C₂₀ alkylene, C₆-C₁₂ phenylene, C₅-C₂₀ cycloalkylene, and C₈-C₂₀ alkylene phenylene groups. Preferably, the alkylene groups should have 2 carbon atoms. Further, the bridging group can be unsubstituted or substituted with C₁-C₂₀ alkyl, alkenyl, benzyl, phenyl and aryl radicals.

The preferred precursors and peroxygen acid derivative compounds are exemplified by structures III and IV.

Within the context of this invention, there may be compounds having the general structure (I) where R₁ and R₄ together or R₁ and R₂ together form an aryl-substituted or unsubstituted, nitrogen-containing heterocylic ring system. Representative of these systems are rings defining pyridine, morpholine, pyrrolidine, piperidine and piperazine. 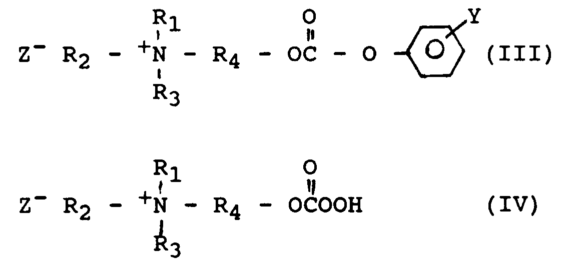
The following compounds are illustrative of precursors within the present invention. It is also to be understood that upon perhydrolysis elimination of the leaving group, as defined above, there remains an organic peroxygen acid derivative of the structures outlined below.
2-(N-benzyl-N,N-dimethylammonium)ethyl sodium 4-sulphophenyl carbonate chloride
2-(N,N,N-trimethylammonium)ethyl sodium 4-sulphophenyl carbonate chloride
2-(N,N-ditallow-N-methylammonium)ethyl sodium 4-sulphophenyl carbonate chloride
3-(N-nonyl-N,N-dimethylammonium)propyl sodium 2-sulphophenyl carbonate chloride
2-(N-benzyl-N,N-diethylammonium)ethyl sodium 2-sulphophenyl carbonate methosulphate
2-(N-benzyl-N,N-dimethylammonium)ethyl disodium 2,4-disulphophenyl carbonate methosulphate
2-(N-butyl-N,N-dimethylammonium)ethyl sodium 4-carboxyphenyl carbonate bromide
2-(N-stearyl-N,N-diethylammonium)ethyl 2-triethanolammoniumphenyl carbonate dichloride
2-(N-diethylhexyl-N-N-dimethylammonium)ethyl 2-(dimethyl amine oxide)phenyl carbonate chloride
2-(N,N,N-triethylammonium)ethyl disodium 2,4-disulphophenyl carbonate methosulphate
4-(N,N,N-trimethylammonium)butyl sodium 4-sulphophenyl carbonate bromide
2-(N,N,N-tributylammonium(ethyl sodium 4-triethanolammoniumphenyl carbonate dichloride
2-(N,N,N-trimethylammonium)ethyl sodium 4-(diethylamine oxide)phenyl carbonate chloride
2-(N,N,N-tribenzylammonium)ethyl 4-carboxyphenyl carbonate methosulphate
1-(N,N-dihexyl-N-methylammonium)-3-phenyl-2-propyl disodium 2,4-disulphophenyl carbonate chloride
2-(N,N,N-tributylammonium)-3-(4-hexylphenyl)-1-propyl sodium 4-sulphophenyl carbonate chloride
6-[(N,N,N-triethylammonium)methyl]-6-dodecyl sodium carboxyphenyl carbonate chloride
2-(N,N-didodecyl-N-ethylammonium)propyl sodium 4-sulphophenyl carbonate chloride
2-[N-benzyl-N-(2-hydroxyethyl)-N-dodecylammonium]ethyl sodium 4-sulphophenyl carbonate chloride
2-(N-decyl-N,N-diethylammonium)ethyl 4-sulphophenyl sodium carbonate chloride
4-(N-phenyl-N,N-didodecylammonium)butyl sodium 4-sulphophenyl carbonate chloride
5-(N-dodecyl-N,N-dimethylammonium)-6-dodecyl sodium 4-sulphophenyl carbonate chloride
2-[2-dodecyl-4-(N,N,N-triethylammonium)phenyl]ethyl sodium 4-sulphophenyl carbonate chloride
Sodium N-[2-(4-sulphophenoxycarbonyloxy)ethyl]-4-decylpyridinium chloride
Sodium N-[2-(4-sulphophenoxycarbonyloxy)ethyl]imidazolium chloride
Disodium bis[(4-sulphophenoxycarbonyloxy)ethyl]methyldodecyl ammonium chloride
Trisodium tris[(4-sulphophenoxycarbonyloxy)ethyl]dodecyl ammonium chloride
2-(N,N,N-trimethylammonium)tetradecyl sodium 4-sulphophenyl carbonate chloride
2-(N-octyl-N,N-dimethylammonium)ethyl sodium 4-sulphophenyl carbonate chloride
2-(N,N-didecyl-N-methylammonium)ethyl sodium 4-sulphophenyl carbonate chloride
2-(N-benzyl-N-dodecyl-N-methylammonium)ethyl sodium 4-sulphophenyl carbonate chloride
2-(N,N,N-trioctylammonium)ethyl sodium 4-sulphophenyl carbonate chloride
1-(N,N,N-trimethylammonium)-2-dodecyl sodium 4-sulphophenyl carbonate chloride
1-(N-benzyl-N,N-diethylammonium)-3-dodecyl sodium 4-sulphophenyl carbonate chloride
1-(N-benzyl-N,N-dibutylammonium)-2-octyl sodium 4-carboxyphenyl carbonate chloride
2-(N,N,N-trihexylammonium)-1-phenylethyl 4-(dimethylamine oxide) phenyl carbonate chloride
12-(N,N,N-triethylammonium)dodecyl 4-triethanolammoniumphenyl carbonate dichloride
2-(N-hexyl-N,N-dimethylammonium)ethyl sodium 4-sulphophenyl carbonate methosulphate
2-(benzyldimethylphosphonium)ethyl sodium 4-sulphophenyl carbonate chloride
2-(trimethylphosphonium)ethyl sodium 4-sulphophenyl carbonate chloride
2-(ditallowmethylphosphonium)ethyl sodium 4-sulphophenyl carbonate chloride
3-(nonyldimethylphosphonium)propyl sodium 2-sulphophenyl carbonate chloride
2-(benzyldiethylphosphonium)ethyl sodium 2-sulphophenyl carbonate methosulphate
2-(benzyldimethylphosphonium)ethyl disodium 2,4-disulphophenyl carbonate methosulphate
2-(butyldimethylphosphonium)ethyl sodium 4-carboxyphenyl carbonate bromide
2-(stearyldiethylphosphonium)ethyl 2-triethanol ammoniumphenyl carbonate dichloride
2-(diethylhexyldimethylphosphonium)ethyl 2-(dimethyl amine oxide)phenyl carbonate chloride
2-(triethylphosphonium)ethyl disodium 2,4-disulphophenyl carbonate methosulphate
4-(trimethylphosphonium)butyl sodium 4-sulphophenyl carbonate bromide
2-(tributylphosphonium)ethyl sodium 4-triethanol ammoniumphenyl carbonate dichloride
2-(trimethylphosphonium)ethyl 4-(diethylamine oxide)phenyl carbonate chloride
2-(tribenzylphosphonium)ethyl sodium 4-carboxyphenyl carbonate methosulphate
1-(dihexyl methylphosphonium)-3-phenyl-2-propyl disodium 2,4-disulphophenyl carbonate chloride
2-(tributylphosphonium)-3-(4-hexylphenyl)-1-propyl sodium 4-sulphophenyl carbonate chloride
6-[(triethylphosphonium)methyl]-6-dodecyl sodium carboxyphenyl carbonate chloride
2-(didodecyl ethylphosphonium) propyl sodium 4-sulphophenyl carbonate chloride
2-[benzyl (2-hydroxyethyl)dodecylphosphonium]ethyl sodium 4-sulphophenyl carbonate chloride
2-(decyl diethylphosphonium)ethyl 4-sulphophenyl sodium carbonate chloride
4-(phenyl didodecylphosphonium)butyl sodium 4-sulphophenyl carbonate chloride
5-(dodecyl dimethylphosphonium)-6-dodecyl sodium 4-sulphophenyl carbonate chloride
2-[2-dodecyl-4-(triethylphosphonium)phenyl]ethyl sodium 4-sulphophenyl carbonate chloride
Disodium bis[(4-sulphophenoxycarbonyloxy)ethyl]methyldodecyl phosphonium chloride
Trisodium tris[4-sulphophenoxycarbonyloxy)ethyl]dodecyl phosphonium chloride
2-(trimethylphosphonium)tetradecyl sodium 4-sulphophenyl carbonate chloride
2-(octyl dimethylphosphonium)ethyl sodium 4-sulphophenyl carbonate chloride
2-(didecyl methylphosphonium)ethyl sodium 4-sulphophenyl carbonate chloride
2-(benzyl dodecyl methylphosphonium)ethyl sodium 4-sulphophenyl carbonate chloride
2-(trioctylphosphonium)ethyl sodium 4-sulphophenyl carbonate chloride
1-(trimethylphosphonium)-2-dodecyl sodium 4-sulphophenyl carbonate chloride
1-(benzyl diethylphosphonium)-3-dodecyl sodium 4-sulphophenyl carbonate chloride
1-(benzyl dibutylphosphonium)-2-octyl sodium 4-carboxyphenyl carbonate chloride
2-(trihexylphosphonium)-1-phenylethyl 4-(dimethylamine oxide) phenyl carbonate chloride
12-(triethylphosphonium)dodecyl 4-triethanolammonium phenyl carbonate dichloride
2-(hexyl dimethylphosphonium)ethyl sodium 4-sulphophenyl carbonate methosulphate
Precursors of the present invention represent a new class of quaternary ammonium- and phosphonium-substituted peroxy carbonic acid bleaches. The precursors described by structure (I) generate the corresponding percarbonic acids rapidly in the presence of hydrogen peroxide or hydrogen peroxide generating persalts such as sodium perborate. Outstanding bleaching is achieved on hydrophilic stains such as tea and red wine. Effective bleaching of tea and red wine stains may occur as low as 20°C and even be perceptive at 10°C. Good bleaching is obtained even at a low molar ratio of hydrogen peroxide to precursor (as low as 1:1) or at a low theoretical percarbonic acid level (5 ppm active oxygen). Typically, the ratio of hydrogen peroxide (or a peroxygen compound generating the equivalent amount of H₂O₂) to precursor will range from 0.5:1 to 10:1, preferably 1:1 to 4:1, most preferably 1:1 to less than 1.5:1. Hydrophobic type stains such as that imparted by spaghetti sauce may even successfully be attacked by appropriate members of the herein disclosed peroxy carbonic acid class. Thus, the precursors of the invention provide effective colour safe, cold water bleaching systems.

Although not to be bound by any theory, it is believed that the quaternary ammonium or phosphonium group enhances the interaction between bleach and the negatively charged fabric surface in detergent solution. Moreover, it is believed that the higher electrophilicity of the peroxy carbonic relative to the peroxy carboxylic type acid functions to increase oxidative power against stains. Thus, peroxy carbonic acid and ester precursors are performance distinguished from known systems such as described in US Patent 4,397,757 and US Patent 4,412,934.

The foregoing precursors may be incorporated in detergent bleach compositions which require as an essential component a peroxygen bleaching compound capable of yielding hydrogen peroxide in an aqueous solution.

Hydrogen peroxide sources are well known in the art. They include the alkali metal peroxides, organic peroxide bleaching compounds such as urea peroxide, and inorganic persalt bleaching compounds, such as the alkali metal perborates, percarbonates, perphosphates and persulphates. Mixtures of two or more such compounds may also be suitable. Particularly preferred are sodium perborate tetrahydrate and, especially, sodium perborate monohydrate. Sodium perborate monohydrate is preferred because it has excellent storage stability while also dissolving very quickly in aqueous bleaching solutions. Rapid dissolution is believed to permit formation of higher levels of percarboxylic acid which would enhance surface bleaching performance.

A detergent formulation containing a bleach system consisting of an active oxygen-releasing material and a novel compound of the invention will usually also contain surface-active materials, detergency builders and other known ingredients of such formulations.

The surface-active material may be naturally derived, such as soap, or a synthetic material selected from anionic, nonionic, amphoteric, zwitterionic, cationic actives and mixtures thereof. Many suitable actives are commercially available and are fully described in literature, for example in "Surface Active Agents and Detergents", Volumes I and II, by Schwartz, Perry and Berch. The total level of the surface-active material may range up to 50% by weight, preferably being from about 1% to 40% by weight of the composition, most preferably 4 to 25%.

Synthetic anionic surface-actives are usually water-soluble alkali metal salts of organic sulphates and sulphonates having alkyl radicals containing from about 8 to about 22 carbon atoms, the term alkyl being used to include the alkyl portion of higher aryl radicals.

Examples of suitable synthetic anionic detergent compounds are sodium and ammonium alkyl sulphates, especially those obtained by sulphating higher (C₈-C₁₈) alcohols produced, for example, from tallow or coconut oil; sodium and ammonium alkyl (C₉-C₂₀) benzene sulphonates, particularly sodium linear secondary alkyl (C₁₀-C₁₅) benzene sulphonates; sodium alkyl glyceryl other sulphates, especially those ethers of the higher alcohols derived from tallow or coconut oil and synthetic alcohols derived from petroleum; sodium coconut oil fatty acid monoglyceride sulphates and sulphonates; sodium and ammonium salts of sulphuric acid esters of higher (C₉-C₁₈) fatty alcohol-alkylene oxide, particularly ethylene oxide, reaction products; the reaction products of fatty acids such as coconut fatty acids esterified with isethionic acid and neutralized with sodium hydroxide; sodium and ammonium salts of fatty acid amides of methyl taurine; alkane monosulphonates such as those derived by reacting alphaolefins (C₈-C₂₀) with sodium bisulphite and those derived by reacting paraffins with SO₂ and Cl₂ and then hydrolyzing with a base to produce a random sulphonate; sodium and ammonium C₇-C₁₂ dialkyl sulphosuccinates; and olefin sulphonates, which term is used to describe the material made by reacting olefins, particularly C₁₀-C₂₀ alpha-olefins, with SO₃ and then neutralizing and hydrolyzing the reaction product. The preferred anionic detergent compounds are sodium (C₁₁-C₁₅) alkylbenzene sulphonates, sodium (C₁₆-C₁₈) alkyl sulphates and sodium (C₁₆-C₁₈) alkyl ether sulphates.

Examples of suitable nonionic surface-active compounds which may be used, preferably together with the anionic surface-active compounds, include in particular the reaction products of alkylene oxides, usually ethylene oxide, with alkyl (C₆-C₂₂) phenols, generally 5-25 EO, i.e. 5-25 units of ethylene oxides per molecule; the condensation products of aliphatic (C₈-C₁₈) primary or secondary linear or branched alcohols with ethylene oxide, generally 6-30 EO, and products made by condensation of ethylene oxide with the reaction products of propylene oxide and ethylene diamine. Other so-called nonionic surface-actives include alkyl polyglycosides, long chain tertiary amine oxides, long chain tertiary phosphine oxides and dialkyl sulphoxides.

Amounts of amphoteric or zwitterionic surface-active compounds can also be used in the compositions of the invention but this is not normally desired owing to their relatively high cost. If any amphoteric or zwitterionic detergent compounds are used, it is generally in small amounts in compositions based on the much more commonly used synthetic, anionic and nonionic actives.

As stated above, soaps may also be incorporated in the compositions of the invention, preferably at a level of less than 30% by weight. They are particularly useful at low levels in binary (soap/anionic) or ternary mixtures together with nonionic or mixed synthetic anionic and nonionic compounds. Soaps which are used are preferably the sodium, or less desirably potassium, salts of saturated or unsaturated C₁₀-C₂₄ fatty acids or mixtures thereof. The amount of such soaps can be varied between about 0.5% and about 25% by weight, with lower amounts of about 0.5% to about 5% being generally sufficient for lather control. Amounts of soap between about 2% and about 20%, especially between about 5% and about 15%, are used to give a beneficial effect on detergency. This is particularly valuable in compositions used in hard water when the soap acts as a supplementary builder.

The detergent compositions of the invention will normally also contain a detergency builder. Builder materials may be selected from 1) calcium sequestrant materials, 2) precipitating materials, 3) calcium ion-exchange materials and 4) mixtures thereof.

Examples of calcium sequentrant builder materials include alkali metal polyphosphates, such as sodium tripolyphosphate; nitrilotriacetic acid and its water-soluble salts; the alkali metal salts of carboxymethyloxy succinic acid, ethylene diamine tetraacetic acid, oxydisuccinic acid, mellitic acid, benzene polycarboxylic acids, citric acid; and polyacetalcarboxylates as disclosed in US Patents 4,144,226 and 4,146,495.

Examples of precipitating builder materials include sodium orthophosphate, sodium carbonate and long-chained fatty acid soaps.

Examples of calcium ion-exchange builder materials include the various types of water-insoluble crystalline or amorphous aluminosilicates, of which zeolites are the best known representatives.

In particular, the compositions of the invention may contain any one of the organic or inorganic builder materials, such as sodium or potassium tripolyphosphate, sodium or potassium pyrophosphate, sodium or potassium orthophosphate, sodium carbonate, the sodium salt of nitrilotriacetic acid, sodium citrate, carboxymethyl malonate, carboxymethyloxysuccinate and the water-insoluble crystalline or amorphous aluminosilicate builder materials, or mixtures thereof.

These builder materials may be present at a level of, for example, from 5 to 80% by weight, preferably from 10 to 60% by weight.

When the peroxygen compound and bleach precursor are dispersed in water, a peroxy acid (IV) is generated which should deliver about 0.1 to about 50 ppm active oxygen per liter of water; preferably oxygen delivery should range from 2 to 30 ppm. Surfactant should be present in the wash water from about 0.05 to 2.0 grams per liter, preferably from 0.15 to 0.50 grams per liter. When present, the builder amount will preferably range from about 0.1 to 6.0 grams per liter.

Apart from the components already mentioned, the detergent compositions of the invention can contain any of the conventional additives in the amounts in which such materials are normally employed in fabric washing detergent compositions. Examples of these additives include lather boosters such as alkanolamides, particularly the monoethanolamides derived from palmkernel fatty acids and coconut fatty acids, lather depressants such as alkyl phosphates and silicones, anti-redeposition agents such as sodium carboxymethyl cellulose and alkyl or substituted alkylcellulose ethers, other stabilizers such as ethylene diamine tetraacetic acid, fabric softening agents, inorganic salts such as sodium sulphate, and, usually present in very small amounts, fluorescent agents, perfumes, enzymes such as proteases, cellulases, lipases and amylases, germicides and colourants.

The bleach precursors and their peroxycarbonic acid derivatives described herein are useful in a variety of cleaning products. These include laundry detergents, laundry bleaches, hard surface cleaners, toilet bowl cleaners, automatic dishwashing compositions and even denture cleaners. Precursors of the present invention can be introduced in a variety of product forms including powders, on sheets or other substrates, in pouches, in tablets or in non-aqueous liquids such as liquid nonionic detergents.

The following Examples will more fully illustrate the embodiments of this invention. All parts, percentages and proportions referred to herein and in the appended claims are by weight unless otherwise illustrated.

### EXAMPLE 1

### Preparation of Choline Chloroformate Chloride + [(CH₃)₃NCH₂CH₂OCOCl]Cl⁻

Phosgene (113 g, 1.15 moles) was condensed in a 500 ml three-neck flask equipped with an inlet gas dispersion tube, dropping funnel, magnetic stirring bar, and dry ice/acetone condenser topped with a drying tube. The phosgene was contained in a small cylinder and was introduced via the gas dispersion tube. A dry ice/acetone bath was used to keep the phosgene at -30°C. Thereinto was added 250 ml dry chloroform (dried over anhydrous calcium chloride for 48 hours) by means of a dropping funnel. Dry, pulverized choline chloride (40 g, 0.29 mole; dried in a vacuum oven at ≧50°C for 24 hours) was added thereto. The mixture was stirred rapidly at -30°C for 1 hour and then allowed to warm to room temperature. Eventually, the reaction mixture separated into two layers. Stirring was continued overnight; hydrogen chloride and any phosgene that escaped during this process was directed to two traps containing 1 N sodium hydroxide.

Workup of the reaction mixture was accomplished by removing the dispersion tube and dropping funnel and attaching a single piece distillation unit to the reaction flask. The receiver flask was covered with a blanket of dry ice. All volatiles were removed from the reaction solution by aid of a water aspirator, leaving white, crystalline choline chloroformate chloride. This product was used without further purification.

Attempts were made to obtain the NMR spectrum of choline chloroformate chloride in a variety of solvents. Unfortunately, this compound is soluble only in water, in which decomposition and accompanying decarboxylation interferes severely with spectral quality. As a result, NMR analysis of choline chloroformate could not be reported. However, the infrared spectrum in Nugol showed a representative carbonyl peak at 1765 cm⁻¹.

### Preparation of 2-(N,N,N-Trimethylammonium)ethyl Sodium 4-sulphophenyl Carbonate Chloride (SPCC)

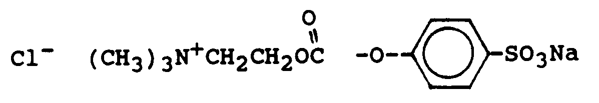

Sodium 4-phenolsulphonate dihydrate (6.4 g, 0.028 mol) and sodium hydroxide (1.1 g, 0.028 mol) were dissolved in 60 ml distilled water. Chloline chloroformate chloride (5.6 g, 0.028 mol) was added while stirring the reaction mixture with a high speed stirrer. After all of the choline chloroformate chloride had dissolved (1-2 minutes), the reaction mixture was frozen in dry ice and freeze-dried. The resulting white solid was analyzed by NMR to be >60 mole % of the desired product (SPCC), the major impurities being choline chloride and unreacted sodium 4-phenolsulphonate.

Alternatively, the reaction mixture can be treated with an equal volume of acetone. Thereby the desired product precipitates from solution.

Unreacted p-phenolsulphonate was removed by boiling the crude SPCC in methanol, followed by filtration and drying. Typically, 50 g SPCC was added to 500 ml dry ethanol. The mixture was boiled and solid SPCC was collected by filtration and dried to give SPCC essentially free of unreacted sodium p-phenolsulphonate (by 60 MHz NMR).
NMR (D₂O, trimethylsilylacetic acid standard) : 3.03 (S, 9H); 3.5-3.8 (m, 4H); 7.23 (d, 2H); 7.77 (d, 2H).

### EXAMPLE 2

### Preparation of 2-(N-benzyl-N,N-dimethylammonium) ethyl Chloroformate Chloride

Phosgene (35 ml, 48.5 g, 0.49 mol) was condensed in apparatus identical with that aforesaid. Dry chloroform (15 ml, dried over anhydrous calcium chloride) was added to the phosgene and the solution held at -30° with a dry ice/acetone bath. Benzyldimethyl-2-hydroxyethyl ammonium chloride (24.6 g, 0.144 mol) in 100 ml dry chloroform was slowly added through the dropping funnel. The reaction mixture was held at -30° until the addition was complete. Thereafter, the reaction mixture was allowed to warm to room temperature and stir overnight.

Workup was carried out as described previously. The yield of crystalline product was 24.6 g (77%). This material was used without further purification.
ir (neat, solid, cm⁻¹): 1784, 1488, 1460, 1414, 1376, 1254, 1219, 1163, 875, 773.

### Preparation of 2-(N-benzyl-N,N-dimethylammonium)ethyl sodium 4-sulphophenyl Carbonate Chloride (SPBDMC)

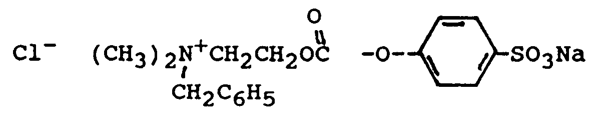

Sodium phenolsulphonate dihydrate (3.94 g, 0.017 mol) and sodium hydroxide (0.68 g, 0.017 mol) were dissolved in distilled water (11 ml) and 2-(N-benzyl-N,N-dimethyl ammonium)-ethyl chloroformate chloride (3.28 g, 0.017 mol) was added while stirring the reaction mixture with a high speed stirrer. After dissolution of the chloroformate, the reaction mixture was quickly diluted to 300 ml with water and freeze-dried. Spectral analysis of the resulting white solid indicated an SPBDMC yield of 47% with unreacted sodium phenolsulphonate and 2-(N-benzyl-N,N-dimethyl-ammonium)ethanol chloride being the principal impurities. The carbonate was used without further purification.
NMR (DMSO/D₂O, trimethylsilylacetic acid standard) : 7.30 (d, 2H); 7.60 (m, 5H); 7.80 (d, 2H); 3.07 (S, 6H).
ir (neat, solid, cm⁻¹): 1766, 1489, 1250, 1212, 1122, 1032, 1010, 704, 616, 567.

### EXAMPLE 3

### Preparation of 2-(N-butyl-N,N-dimethylammonium)ethyl Chloroformate Bromide

This compound was prepared by the procedure described for 2-(N-benzyl-N,N-dimethylammonium)ethyl chloroformate chloride. For this experiment, the reagents were as follows:
2-(N-butyl-N,N-dimethylammonium)ethanol bromide (10.0 g, 5.5 x 10⁻² mol), phosgene (17.5 g, 0.177 mol) and dry chloroform (75 ml). After workup, 2-(N-butyl-N,N-dimethylammonium)ethyl chloroformate chloride was used without further purification. An infrared spectrum of the product (neat) revealed a carbonyl peak at 1770 cm⁻¹.

### Preparation of 2-(N-butyl-N,N-dimethylammonium)ethyl Sodium 4-Sulphophenyl Carbonate Bromide (SPBuDMC)

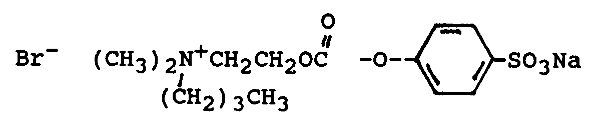

This compound was prepared by the procedure described for 2-(N-benzyl)-N,N-dimethylammonium)ethyl sodium 4-sulphophenyl carbonate bromide. Typical reagent levels were as follows:
2-(N-butyl-N,N-dimethylammonium)ethyl chloroformate bromide (4.03 g, 17.2 x 10⁻² mol), sodium 4-phenol sulphonate dihydrate (4.00 g, 1.72 x 10⁻² mol), sodium hydroxide (0.70 g, 1.75 x 10⁻² mol), and water (8.0 ml).

Spectral analysis of the white, solid product indicated the SPBuDMC yield was 66% with unreacted sodium phenol sulphonate and 2-(N-butyl-N,N-dimethylammonium)ethyl bromide being the principal impurities. These impurities made assignment of aliphatic peaks in the NMR spectrum difficult and, as a result, only the aromatic proton peak positions of the phenolsulphonate group and nitrogen bound methyl group in the product are herein reported.
NMR (D₂O, trimethylsilylacetic acid standard) : 7.7 (d, 2H); 7.2 (d, 2H); 2.9 (5, 6H).

### EXAMPLE 4

### Preparation of 2-[4-N,N,N-trimethylammonium)phenyl]-Ethanol Chloride

Methylene chloride (50 ml) and 2-[4-(N,N-dimethylamino)phenyl]ethanol (5.00 g, 3.03 x 10⁻² mol) were placed in a 100 ml round-bottom flask equipped with a dropping funnel, condensor, and magnetic stirring bar. Methyl iodide (4.29 g, 3.03 x 10⁻² mol) was added dropwise through the dropping funnel. Precipitate began to form immediately. After addition of all of the methyl iodide, the reaction mixture was stirred for an additional 30 minutes. The product was collected by vacuum filtration, washed with methylene chloride, and dried in a vacuum oven. Spectral analysis confirmed the structure of the product as 2-[N,N,N-trimethylammonium)phenyl]ethanol iodide. The iodide salt was converted to the hydroxide salt by passing through a Bio Rad AG21K resin exchanged with sodium hydroxide. Neutralization of the hydroxide salt with dilute hydrochloric acid, followed by freze-drying, gave the desired chloride salts.

### Preparation of 2-[4-(N,N,N-trimethylammonium)phenyl] ethyl Chloroformate Chloride

This compound was prepared by the procedure described for 2-(N-benzyl-N,N-dimethylammonium)ethyl chloroformate chloride. Typical reagent levels were as follows: 2-[4-(N,N,N-trimethylammonium)phenyl]ethanol chloride (4.56 g, 2.12 x 10⁻² mol), phosgene (8.40 g, 8.48 x 10⁻² mol), and dry chloroform (30 ml).

After workup, 2-[4-(N,N,N-trimethylammonium)phenyl] ethyl chloroformate chloride was used without further purification.

### Preparation of 2-[4-(N,N,N-trimethylammonium)phenyl] ethyl Sodium 4-Sulphophenyl Carbonate Chloride (SPTPEC)

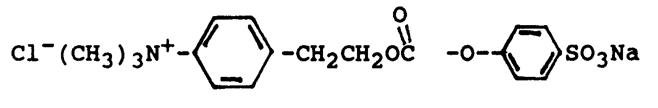

This compound was prepared by the procedure described for 2-(N-benzyl-N,N-dimethylammonium)ethyl sodium 4-sulphophenyl carbonate chloride. Typical reagent levels were as follows:
2-[4-(N,N,N-trimethylammonium)phenyl]ethyl chloroformate chloride (4.10 g, 1.50 x 10⁻² mol), sodium 4-phenolsulphonate dihydrate (2.42 g, 1.50 x 10⁻² mol), sodium hydroxide (0.59 g, 1.50 x 10⁻² mol) and water (6.4 ml).

The product crystallized from the reaction mixture. After drying, spectral analysis confirmed the product structure as 2-[4-(N,N,N-trimethylammonium)phenyl]ethyl sodium 4-sulphophenyl carbonate chloride. Purity was approximately 65% (by NMR).

The product was purified by boiling in methanol, followed by filtration and drying. The NMR spectrum of the purified product showed complete absence of unreacted sodium phenolsulphonate.
NMR (D₂O, trimethylsilylacetic acid standard): 7.55 (d, 2H); 7.45 (d, 2H); 7.20 (d, 2H); 7.00 (d, 2H); 4.30 (t, 2H); 3.35 (s, 9H); 2.85 (t, 2H).
ir (solid, photoacoustic cm⁻¹): 3023, 1755, 1519, 1462, 1151, 1123, 957, 852, 836, 818.

### EXAMPLE 5

### Preparation of 1,1-Dimethyl-3-hydroxypiperidinium Chloride

This compound was prepared by the procedure described for 2-[4-(N,N,N-trimethylammonium)phenyl]ethanol chloride. Typical reagent levels were as follows: 3-hydroxy-1-methylpiperidine (21.7 g, 0.188 mol), iodomethane (40.0 g, 0.280 mol) and methylene chloride (50 ml).
NMR (D₂O, TMS external standard):
4.10 (m, 1H); 3.30 (m, 2H); 3.16 (s, 3H); 3.03 (s, 3H); 2.13-1.16 (m, 4H).

### Preparation of 1,1-Dimethylpiperidium-3-chloroformate Chloride

This compound was prepared by the procedure described for 2-(N-benzyl-N,N-dimethylammonium(ethyl chloroformate chloride. Typical reagent levels were as follows: 1,1-dimethyl-3-hydroxypiperidinium chloride (24.0 g, 0.124 mol), phosgene (41.6 ml, 0.583 mol) and dry chloroform (100 ml).

After workup, 1,1-dimethylpiperidinium-3-chloroformate chloride was used without further purification.

### Preparation of Sodium 3-(1,1-Dimethylpiperidinium) 4-Sulphophenyl Carbonate Chloride (SPDPC)

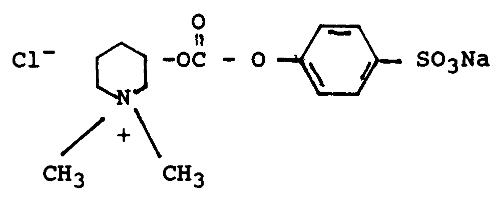

This compound was prepared by the procedure described for 2-(N-benzyl-N,N-dimethylammonium)ethyl sodium 4-sulphophenyl carbonate chloride. Typical reagent levels were as follows:
1,1-dimethylpiperidinium-3-chloroformate chloride (4.65 g, 2.19 x 10⁻² mol), sodium 4-sulphophenol dihydrate (5.10 g, 2.19 x 10⁻² mol), sodium hydroxide (0.88 g, 2.20 x 10⁻² mol), and water (10 ml).

Spectral analysis of the white, solid product indicated the SPDPC yield was approximately 70%, with major impurities being unreacted sodium 4-sulphophenol and 1,1-dimethyl-3-hydroxpiperidinium chloride.
NMR (D₂O, TMS external standard):
7.56 (d, 2H); 7.08 (d, 2H); 9.92 (m, 1H); 3.52-2.96 (m, 4H) 2.86 (s, 3H); 2.83 (s, 3H); 1.72 (m, 4H).

### EXAMPLE 6

### Preparation of 1,1-Dimethyl-4-hydroxypiperidinium Chloride

This compound was prepared by the procedure described for 2-[4-N,N,N-trimethylammonium)phenyl]ethanol chloride. Typical reagent levels were as follows: 4-hydroxy-1-methyl-piperidine (21.7 g, 0.188 mol), iodomethane (40.0 g, 0.280 mol), and methylene chloride (50 ml).
NMR (D₂O, TMS external standard) :
3.96 (m, 1H); 3.40 (m, 4H); 3.12 (s, 6H); 2.00 (m, 4H).

### Preparation of 1,1-Dimethylpiperidinium-4-chloroformate Chloride

This compound was prepared by the procedure described for 2-(N-benzyl-N,N-dimethylammonium)ethyl chloroformate chloride. Typical reagent levels were as follows: 1,1-dimethyl-4-hydroxypiperidinium chloride (24.0 g, 0.145 mol), phosgene (41.6 ml, 0.583 mol), and dry chloroform (100 ml).

After workup, the product was used without further purification.

### Preparation of Sodium 4-(1,1-dimethylpiperidinium) 4-sulphophenyl Carbonate Chloride (SPDMPC)

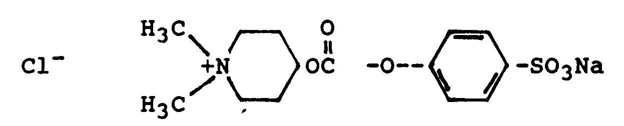

This compound was prepared by the procedure described for 2-(N-benzyl-N,N-dimethylammonium)ethyl sodium 4-sulphophenyl carbonate chloride. Typical reagent levels were as follows:
1,1-dimethylpiperidinium-4-chloroformate chloride (4.65 g, 2.19 x 10⁻² mol), sodium 4-sulphophenol dihydrate (5.10 g, 2.19 x 10⁻² mol), sodium hydroxide (0.88 g, 2.20 x 10⁻² mol), and water (10 ml).

The white, solid product was purified by boiling in ethanol, followed by filtration and drying to give a solid containing no unreacted sodium 4-sulphophenol nor 1,1-dimethyl-4-hydroxypiperidinium chloride by NMR analysis.
NMR (D₂O, trimethylsilylacetic acid standard): 7.75 (d, 2H); 7.22 (d, 2H); 5.10 (m, 1H); 3.44 (m, 4H); 3.14 (s, 3H); 3.10 (s, 3H); 2.24 (m, 4H).

### EXAMPLE 7

### Preparation of 2-(N,N,N-trimethylammonium)ethyl 4-Nitrophenyl Carbonate Chloride (STNC)

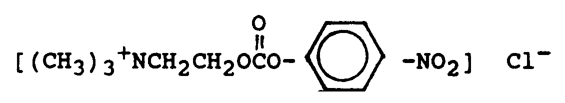

This compound was prepared by the procedure described for 2-(N-benzyl-N,N-dimethylammonium)ethyl sodium 4-sulphophenyl carbonate bromide. Typical reagent levels were as follows:
2-(N,N,N-trimethylammonium)ethyl chloroformate chloride (7.0 g, 3.5 x 10⁻² mol), 4-nitrophenol (4.8 g, 3.5 x 10⁻² mol), 4-nitrophenol (4.8g, 3.5 x 10⁻² mol), sodium hydroxide (1.4 g, 3.5 x 10⁻² mol), and water (15 ml).

Spectral analysis of the white solid indicated the product yield was greater than 90% with 4-nitrophenol and choline chloride being the principal impurities. The product was used without further purification.
NMR (D₂O, TMS external standard): 3.5-3.8 (m, 4H); 3.05 (s, 9H); 7.23 (d, 2H); 8.18 (d, 2H).

### EXAMPLE 8

### Peracid Generation From Precursors

Peroxycarbonic acid precursors described herein can be used to generate peroxycarbonic acid bleaches in basic aqueous solution containing a source of hydrogen peroxide and, optimally, may contain typical detergent ingredients. Peroxycarbonic acid generation was demonstrated by adding a premeasured sample of precursor to 500 ml aqueous buffer solution at the desired pH, heated to 40° in a thermojacketed beaker, and containing the approximate level of hydrogen peroxide (added as either 30% hydrogen peroxide or sodium perborate monohydrate). The hydrogen peroxide source was added just prior to addition of the precursor. The milliliter aliquots of solution were withdrawn from the beaker at regular intervals and were added to a 250 ml titration flask containing crushed ice (150 g), glacial acetic acid (30 ml) and 40% aqueous potassium iodide (5 ml). After development for ten minutes with occasional agitation, the iodine produced was titrated with standard sodium thiosulphate solution. Time zero was taken as the point of introduction of precursor into the peroxide solution. Precursor perhydrolysis experiments were generally carried out for a maximum of 15 minutes.

Since hydrogen peroxide itself contributes to the total active oxygen in these titrations, controls or "blanks" were obtained by carrying out a perhydrolysis experiment in the absence of precursor. These hydrogen peroxide blanks were substracted from the total active oxygen titration in the presence of bleach precursor to give the level of active oxygen produced by precursor perhydrolysis.

Peroxycarbonic acid generation was determined at pH 8, 9, and 10. Borax buffer was used for experiments at pH 9 and 10 while phosphate buffer was employed for experiments carried out at pH 8. Adjustment of the buffer systems at 40°C to the exact pH was carried out with 1 M hydrochloric acid or sodium hydroxide solution.

Tables I and II list the peroxycarbonic acid yields as a percent of theoretical form SPCC and SPBCMC, respectively.

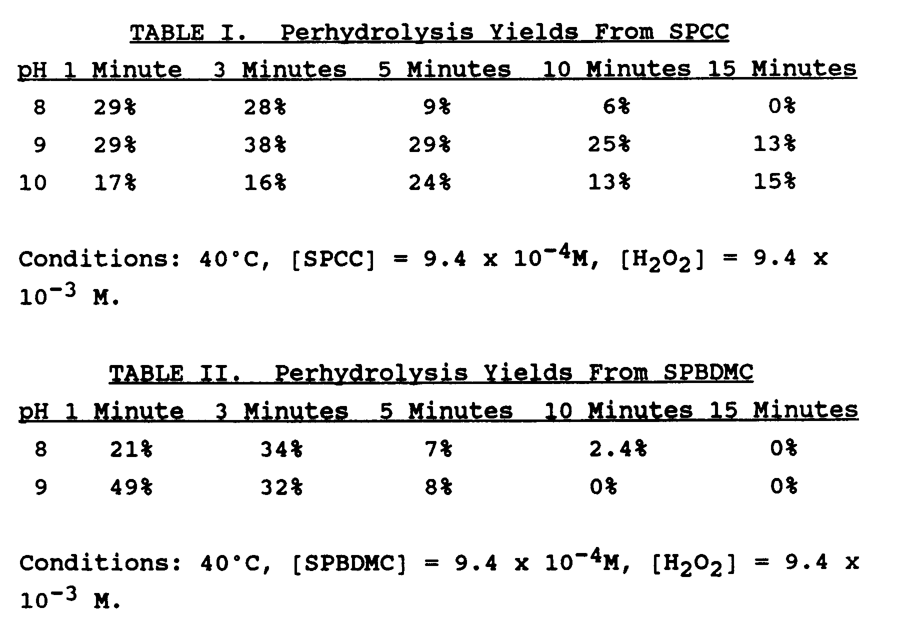

From the data in Tables I and II, it can be seen that precursors SPCC and SPBDMC generate peroxycarbonic acid rapidly. Peracid is generated quickly, even at pH 8.

Peroxycarbonic acid decomposition during the perhydrolysis results in less than quantitative yields based on precursor level.

### EXAMPLE 9

### Bleaching From Peroxycarbonic Acid Precursor/Peroxide Systems

The stain bleaching ability of peroxycarbonic acids generated from the synthesized precursors was demonstrated on common stains such as tea, red wine, and blackberry juice. Typically, cotton test pieces (10 cm x 10 cm) stained with the appropriate stain were washed in a Terg-O-Tometer in 1 l of aqueous solution containing a given level of bleach precursor, hydrogen peroxide, buffer, and surfactant (generally sodium dodecylbenzene sulphonate).

Washes were carried out at 40°C for 15 minutes. Stain bleaching was measured reflectometrically using a Colorgard System/05 Reflectometer. Bleaching is indicated by an increase in reflectance, reported as ΔR. In general, a ΔR of one unit is perceivable in a paired comparison while ΔR of two units is perceivable monadically. In reporting the reflectance change, the change in reflectance caused by general detergency and bleaching by the excess hydrogen peroxide has been accounted for. Thus ΔR can actually be expressed as:

$\text{ΔR = (Reflectance of stained fabric washed with precursor/H₂O₂ and detergent - Reflectance of stained fabric before washing) - (Reflectance of stained fabric washed with H₂O₂ and detergent alone - Reflectance of stained fabric before washing).}$

In the case of spaghetti stain, bleaching is measured as "Δb" where the quantity "Δb" is the change in the b-axis of the Hunter colour scale. The spaghetti stain is initially yellow and loses colour with bleaching and thus bleaching produces a negative change in b. Since peroxide-only controls were also carried out with the spaghetti sauce stains, percarbonic acid bleaching is actually reported as "Δb".

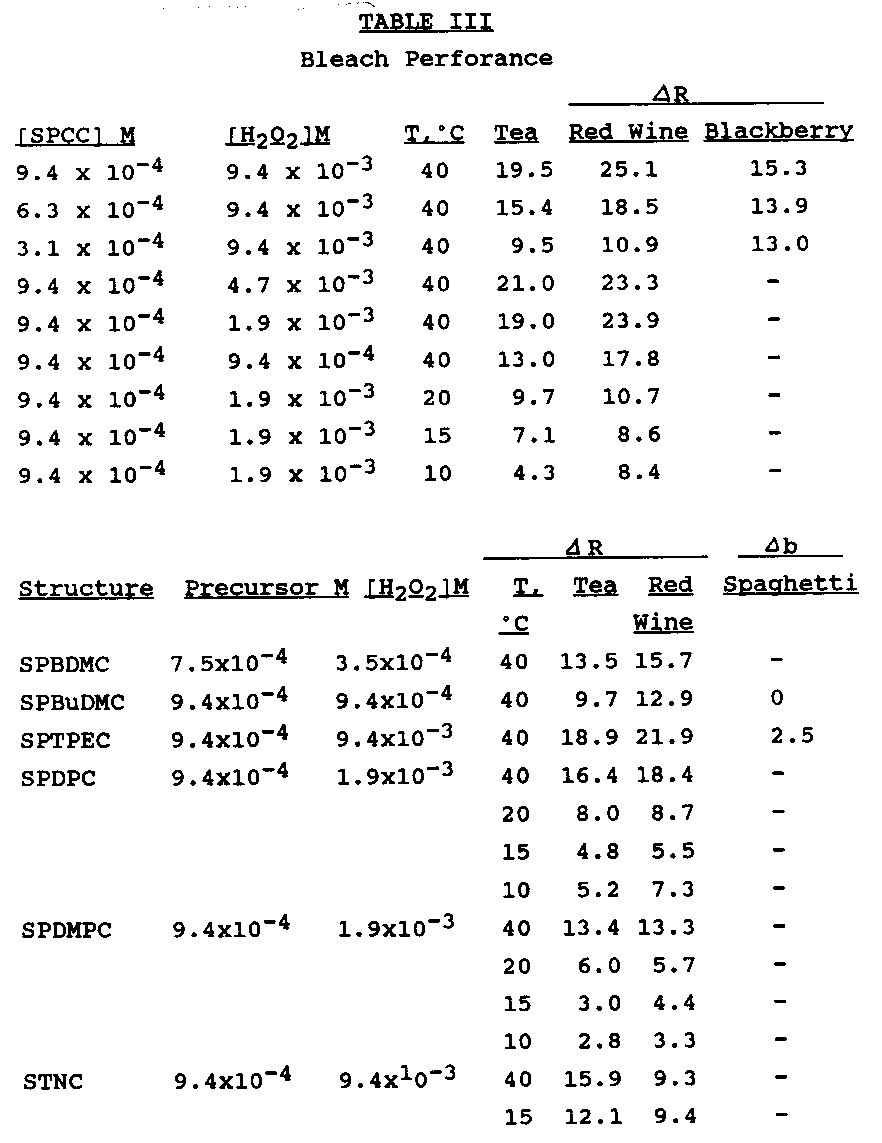

It can be seen that bleaching from these peroxycarbonic acid bleaches is excellent, giving substantial stain removal on a variety of stains. As evidenced from Table, the SPCC system has been studied most extensively. A number of observations may be gleaned from the Table with respect to SPCC. At a theoretical percarbonic acid yield of 15 ppm active oxygen (9.4 x 10⁻⁴ M), outstanding bleaching is obtained at 40°C in 15 minutes on hydrophilic stains such as tea, red wine and blackberry. Bleaching remains outstanding at hydrogen peroxide/precursor ratios as low as 2:1. Even at 1:1, bleaching is very good compared to state-of-the-art systems such as sodium nonanoyloxybenzene sulphonate with perborate. At a theoretical percarbonic acid yield of 5 ppm active oxygen (3.1 x 10⁻⁴ M), bleaching of hydrophlic stains is comparable to that obtained with sodium nonanoyloxybenzene sulphonate with perborate at 10 ppm active oxygen theoretical peracid. Levels of 15 ppm active oxygen give very good bleaching at 20°C and perceivable bleaching even as low as 10°C.

Precursors other than SPCC all gave very good to outstanding bleaching on tea and red wine stains at 40°C and 15 ppm active oxygen theoretical percarbonic acid yield. Most interestingly, SPTPEC gave a modest but perceptible bleaching on spaghetti sauce stain. The observation is unusual in that this stain is hydrophobic whereas the class is most effective against hydrophilic stains. Equally interesting is the observation that SPDPC and SPDMPC are effective in cold water. These results indicate that low temperature bleaching is a general property of percarbonic acid substituted with quaternary ammonium functionality.

The foregoing description and examples illustrate selected embodiments of the present invention.

## Claims

1. A bleach precursor compound having the formula: 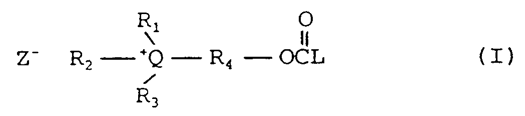 wherein:
R₁, R₂ and R₃ are each a radical selected from the group consisting of alkyl, alkenyl, alkynyl, cycloalkyl, cycloalkenyl, alkaryl, aryl, phenyl, hydroxyalkyl, polyoxyalkylenyl and R₄OCOL;
or two or more of R₁, R₂ and R₃ together form an alkyl-substituted or unsubstituted, nitrogen-containing heterocyclic ring system;
or at least one of R₁, R₂ and R₃ is attached to R₄ to form an alkyl-substituted or unsubstituted, nitrogen-containing heterocyclic ring system;
R₄ is selected from a bridging group consisting of alkylene, cycloalkylene, alkylenephenylene, phenylene, arylene, and polyalkoxylene and wherein the bridging group can be unsubstituted or substituted with C₁-C₂₀ alkyl, alkenyl, benzyl, phenyl and aryl radicals;
Z⁻ is a monovalent or multivalent anion leading to charge neutrality when combined with Q⁺ in the appropriate ratio and wherein Z⁻ is sufficiently oxidatively stable not to interfere significantly with bleaching by a peroxy carbonic acid;
Q is nitrogen or phosphorus; and
L is selected from the group consisting of: 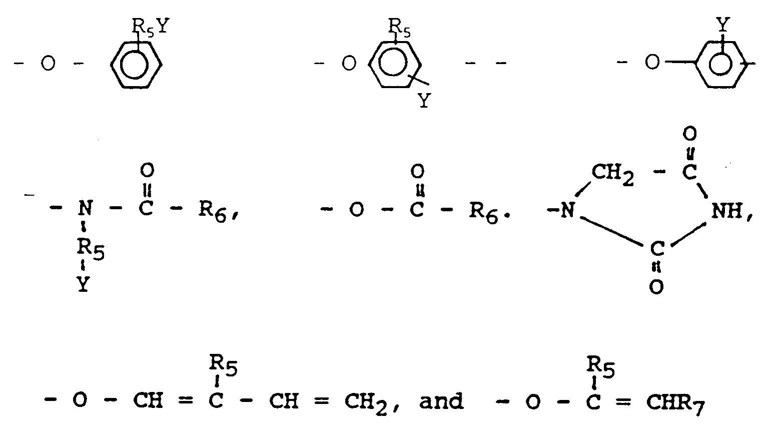 wherein:
R₅ is a C₁-C₁₂ alkyl or alkylene group, R₆ is a C₁-C₁₂ alkyl group, R₇ is H or R₅, and Y is H or a solubilizing group, selected from -SO⁻₃M⁺, -COO⁻M⁺, -SO⁻₄M⁺, -N⁺(R₅)₃X⁻,-NO₂,-OH and O←N(R₅)₂ and mixtures thereof; M⁺ is a hydrogen, alkali metal, ammonium or alkyl or hydroxyalkyl-substituted ammonium cation and X⁻ is a halide, hydroxide, phosphate, sulphate, methyl sulphate or acetate anion.

2. The precursor of Claim 1 wherein L has the formula: 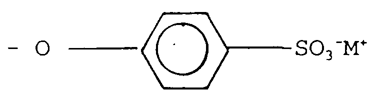 wherein M⁺ is a sodium, potassium or ammonium cation.

3. The precursor of Claim 1 wherein Q is nitrogen and R₁, R₂ and R₃ are each the same or different and selected from C₁-C₂₀ atom radicals selected from the group consisting of alkyl, alkylaryl, benzyl, hydroxyalkyl, and heterocyclic rings containing the quaternary nitrogen where R₁ and R₄ or R₁ and R₂ are joined together, and mixtures of groups thereof.

4. The precursor of Claim 3 wherein R₁ is selected from short-chain C₁-C₄ alkyl radicals.

5. The precursor of Claim 4 wherein R₂ and R₃ are each a longer chain C₇-C₂₀ alkyl or alkylaryl radical.

6. The precursor of Claim 5 wherein said longer chain radical is selected from the group consisting of benzyl, lauryl and stearyl groups.

7. The precursor of Claims 1-4 wherein R₄ is selected from a bridging group consisting of C₂-C₂₀ alkylene, C₆-C₁₂ phenylene, C₅-C₂₀ cycloalkylene, and C₈-C₂₀ alkylenephenylene groups.

8. The precursor of Claim 3 wherein said heterocyclic ring is selected from pyridine, morpholine, pyrrolidone, piperidine and piperazine.

9. The precursor of Claim 1 wherein Y is a sulphonic acid salt.

10. The precursor of Claim 1 wherein the compound is 2-(N,N,N-trimethylammonium) ethyl 4-sulphophenyl carbonate salt.

11. The precursor of Claim 1 wherein the compound is 2-(N-benzyl-N,N-dimethylammonium) ethyl 4-sulphophenyl carbonate salt.

12. The precursor of Claim 1 wherein the compound is 2-(N-butyl-N,N-dimethylammonium) ethyl 4-sulphophenyl carbonate salt.

13. The precursor of Claim 1 wherein the compound is 2-[4-(N,N,N-trimethylammonium) phenyl] ethyl 4-sulphophenyl carbonate salt.

14. The precursor of Claim 1 wherein the compound is 3-(1,1-dimethylpiperidinium) 4-sulphophenyl carbonate salt.

15. The precursor of Claim 1 wherein the compound is 4-(1,1-dimethylpiperidinium) 4-sulphophenyl carbonate salt.

16. A bleaching-detergent composition comprising:
(i) from 1 to 60% of a peroxygen compound capable of yielding hydrogen peroxide in an aqueous solution;
(ii) from 0.1 to 40% of a bleach precursor having the formula: 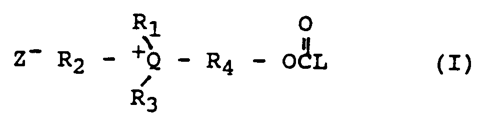
wherein:
R₁, R₂ and R₃ are each a radical selected from the group consisting of alkyl, alkenyl, alkynyl, cycloalkyl, cycloalkenyl, alkaryl, aryl, phenyl, hydroxyalkyl, polyoxyalkylenyl and R₄OCOL;
or two or more of R₁, R₂, and R₃ together form an alkyl-substituted or unsubstituted, nitrogen-containing heterocyclic ring system;
or at least one of R₁, R₂ and R₃ is attached to R₄ to form an alkyl-substituted or unsubstituted, nitrogen-containing heterocyclic ring system;
R₄ is selected from a bridging group consisting of alkylene, cycloalkylene, alkylenephenylene, phenylene, arylene, and polyalkoxylene and wherein the bridging group can be unsubstituted or substituted with C₁-C₂₀ atoms selected from alkyl, alkenyl, benzyl, phenyl and aryl radicals;
Z⁻ is a monovalent or multivalent anion leading to charge neutrality when combined with Q⁺ in the appropriate ratio and wherein Z⁻ is sufficiently oxidatively stable not to interfere significantly with bleaching by a peroxy carbonic acid;
Q is nitrogen or phosphorus; and
L is selected from the group consisting of 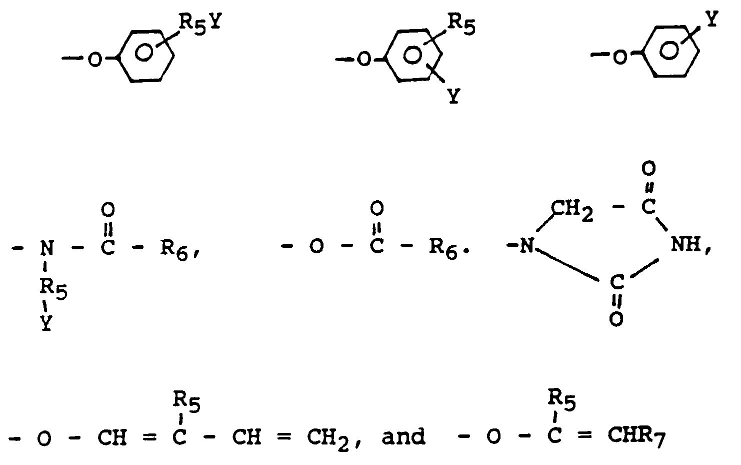 wherein:
R₅ is a C₁-C₁₂ alkyl or alkylene group, R₆ is a C₁-C₁₂ alkyl group, R₇ is H or R₅, and Y is H or a solubilizing group, selected from -SO⁻₃M⁺, -COO⁻M⁺, -SO⁻₄M⁺, -N⁺(R₅)₃X⁻,-NO₂, -OH, and O ←N(R₅)₂ and mixtures thereof; M⁺ is a hydrogen, alkali metal, ammonium or alkyl or hydroxyalkyl-substituted ammonium cation. X⁻ is a halide, hydroxide, phosphate, sulphate, methyl sulphate or acetate anion;
(iii) from 0 to 50% of a surfactant; and
(iv) from 5 to 80% of a detergent builder.

17. The composition of Claim 16 wherein the surfactant ranges from 1 to 40% and the detergent builder ranges from 5 to 80% by weight.

18. The composition of Claim 16 wherein L has the formula: 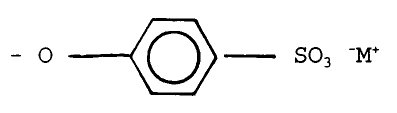 wherein M⁺ is a sodium, potassium or ammonium cation.

19. The composition of Claim 16 wherein Q is nitrogen and R₁, R₂ and R₃ are each the same or different and selected from C₁-C₂₀ atom radicals selected from the group consisting of alkyl, alkylaryl, benzyl, hydroxyalkyl, and heterocyclic rings containing the quaternary nitrogen where R₁ and R₄ or R₁ and R₂ are joined together, and mixtures of groups thereof.

20. The composition of Claim 19 wherein R₁ is selected from short-chain C₁-C₄ alkyl radicals.

21. The composition of Claim 20 wherein R₂ and R₃ are each a longer chain C₇-C₂₀ alkyl or alkylaryl radical.

22. The composition of Claim 21 wherein said longer chain radical is selected from the group consisting of benzyl, lauryl and stearyl groups.

23. The composition of Claim 16 wherein R₄ is selected from a bridging group consisting of C₂-C₂₀ alkylene, C₆-C₁₂ phenylene, C₅-C₂₀ cycloalkylene, and C₈-C₂₀ alkylenephenylene groups

24. The composition of Claim 19 wherein said heterocyclic ring is selected from pyridine, morpholine, pyrrolidone, piperidine and piperazine.

25. The composition of Claim 16 wherein Y is a sulphonic acid salt.

26. The composition of Claim 16 wherein the precursor is 2-(N,N,N-trimethylammonium) ethyl sodium 4-sulphophenyl carbonate salt.

27. The composition of Claim 16 wherein the precursor is 2-(N-benzyl-N,N-dimethylammonium) ethyl sodium 4-sulphophenyl carbonate salt.

28. The composition of Claim 16 wherein the precursor is 2-(N-butyl-N,N-dimethylammonium) ether sodium 4-sulphophenyl carbonate salt.

29. The composition of Claim 16 wherein the precursor is 2-[4-(N,N,N-trimethylammonium) phenyl] ethyl sodium 4-sulphophenyl carbonate salt.

30. The composition of Claim 16 wherein the precursor is sodium 3-(1,1-dimethypiperidinium) 4-sulphophenyl carbonate salt.

31. The composition of Claim 16 wherein the precursor is sodium 4-(1,1-dimethylpiperidinium) 4-sulphophenyl carbonate salt.

## Patentansprüche

1. Eine Bleichmittelvorläuferverbindung mit der Formel: 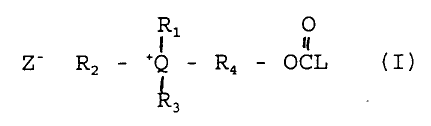 worin:
R₁, R₂ und R₃ jeweils ein Rest sind, ausgewählt aus der Gruppe, die besteht aus Alkyl, Alkenyl, Alkinyl, Cycloalkyl, Cycloalkenyl, Alkaryl, Aryl, Phenyl, Hydroxyalkyl, Polyoxyalkylenyl und R₄OCOL;
oder zwei oder mehr von R₁, R₂ und R₃ zusammen ein Alkyl-substituiertes oder unsubstituiertes, stickstoffhaltiges heterocyclisches Ringsystem bilden;
oder wenigstens einer von R₁, R₂ und R₃ an R₄ gebunden ist, wobei ein alkylsubstituiertes oder unsubstituiertes, stickstoffhaltiges heterocyclisches Ringsystem gebildet wird;
R₄ ausgewählt wird aus einer Brückengruppe, bestehend aus Alkylen, Cycloalkylen, Alkylenphenylen, Phenylen, Arylen und Polyalkoxylen, und wobei die Brückengruppe unsubstituiert oder mit C₁-C₂₀ Alkyl-, Alkenyl-, Benzyl-, Phenyl- und Arylresten substituiert sein kann;
Z⁻ ein monovalentes oder multivalentes Anion ist, das zur Ladungsneutralität bei Kombination mit Q⁺ im geeigneten Verhältnis führt, und wobei Z⁻ ausreichend oxidationsstabil ist, um beim Bleichen durch eine Peroxycarbonsäure nicht signifikant Wechselwirkungen zu zeigen;
Q Stickstoff oder Phosphor ist; und
L ausgewählt aus der Gruppe wird, die besteht aus: 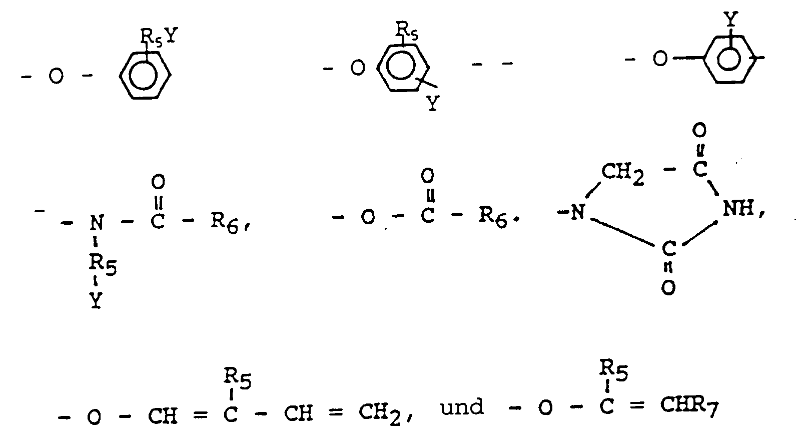 worin:
R₅ eine C₁-C₁₂ Alkyl- oder Alkylengruppe ist, R₆ eine C₁-C₁₂ Alkylgruppe ist, R₇ H oder R₅ ist, und Y H oder eine löslich machende Gruppe ist, ausgewählt aus -SO⁻₃M⁺, -COO⁻M⁺, -SO₄⁻M⁺, -N⁺(R₅)₃X⁻, -NO₂, -OH und O ← N(R₅)₂ und Mischungen davon; worin M⁺ ein Wasserstoff, ein Alkalimetall, Ammonium oder Alkyl- oder Hydroxyalkylsubstituiertes Ammoniumkation ist, X⁻ ein Halogenid, Hydroxid, Phosphat, Sulfat, Methylsulfat oder Acetatanion ist.

2. Der Vorläufer nach Anspruch 1, worin L die Formel besitzt: 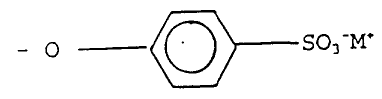 worin M⁺ ein Natrium, Kalium oder Ammoniumkation ist.

3. Der Vorläufer nach Anspruch 1, worin Q Stickstoff ist, und R₁, R₂ und R₃ jeweils gleich oder verschieden sind und ausgewählt werden aus C₁-C₂₀ Atomresten, ausgewählt aus der Gruppe, die besteht aus Alkyl, Alkylaryl, Benzyl, Hydroxyalkyl und heterocyclischen Ringen, die den quarternären Stickstoff enthalten, wo R₁ und R₄ oder R₁ und R₂ miteinander verbunden sind, und Mischung von Gruppen davon.

4. Der Vorläufer nach Anspruch 3, worin R₁ ausgewählt wird aus kurzkettigen C₁-C₄ Alkylresten.

5. Der Vorläufer nach Anspruch 4, worin R₂ und R₃ jeweils ein langkettiger C₇-C₂₀ Alkyl- oder Alkylarylrest sind.

6. Der Vorläufer nach Anspruch 5, worin der längerkettige Rest ausgewählt wird aus der Gruppe, die besteht aus Benzyl, Lauryl und Stearylgruppen.

7. Der Vorläufer nach Ansprüchen 1 bis 4, worin R₄ ausgewählt wird aus einer Brückengruppe, die besteht aus C₂-C₂₀ Alkylen, C₆-C₁₂ Phenylen, C₅-C₂₀ Cycloalkylen und C₈-C₂₀ Alkylenphenylengruppen.

8. Der Vorläufer nach Anspruch 3, worin der heterocyclische Ring aus Pyridin, Morpholin, Pyrrolidon, Piperidin und Piperazin ausgewählt wird.

9. Der Vorläufer nach Anspruch 1, worin Y ein Sulfonsäuresalz ist.

10. Der Vorläufer nach Anspruch 1, worin die Verbindung 2-(N,N,N-Trimethylammonium)ethyl-4-sulfophenylcarbonatsalz ist.

11. Der Vorläufer nach Anspruch 1, worin die Verbindung 2-(N-Benzyl-N,N-dimethylammonium)ethyl-4-sulfophenylcarbonatsalz ist.

12. Der Vorläufer nach Anspruch 1, worin die Verbindung 2-(N-Butyl-N,N-dimethylammonium)ethyl-4-sulfophenylcarbonat salz ist.

13. Der Vorläufer nach Anspruch 1, worin die Verbindung 2-[4-(N,N,N-Trimethylammonium)phenyl]ethyl-4-sulfophenylcarbonatsalz ist.

14. Der Vorläufer nach Anspruch 1, worin die Verbindung 3-(1,1-Dimethylpiperidinium)-4-sulfophenylcarbonatsalz ist.

15. Der Vorläufer nach Anspruch 1, worin die Verbindung 4-(1,1-Dimethylpiperidinium)-4-sulfophenylcarbonatsalz ist.

16. Eine Bleich-Reinigungsmittelzusammensetzung, die umfasst:
i) von 1 bis 60% einer Persauerstoffverbindung, die in der Lage ist, Wasserstoffperoxid in einer wässrigen Lösung zu ergeben;
ii) von 0.1 bis 40% eines Bleichmittelvorläufers mit der Formel: 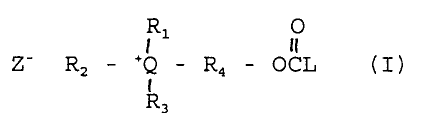
worin:
R₁, R₂ und R₃ jeweils ein Rest sind, ausgewählt aus der Gruppe, die besteht aus Alkyl, Alkenyl, Alkinyl, Cycloalkyl, Cycloalkenyl, Alkaryl, Aryl, Phenyl, Hydroxyalkyl, Polyoxyalkylenyl und R₄OCOL;
oder zwei oder mehr von R₁, R₂ und R₃ zusammen ein Alkylsubstituiertes oder unsubstituiertes, stickstoffhaltiges heterocyclisches Ringsystem bilden;
oder wenigstens einer von R₁, R₂ und R₃ an R₄ gebunden ist, wobei ein alkylsubstituiertes oder unsubstituiertes, stickstoffhaltiges heterocyclisches Ringsystem gebildet wird;
R₄ ausgewählt wird aus einer Brückengruppe, bestehend aus Alkylen, Cycloalkylen, Alkylenphenylen, Phenylen, Arylen und Polyalkoxylen, und wobei die Brückengruppe unsubstituiert oder mit C₁-C₂₀ Alkyl-, Alkenyl-, Benzyl-, Phenyl- und Arylresten substituiert sein kann;
Z⁻ ein monovalentes oder multivalentes Anion ist, das zur Ladungsneutralität bei Kombination mit Q⁺ im geeigneten Verhältnis führt, und wobei Z⁻ ausreichend oxidationsstabil ist, um beim Bleichen durch eine Peroxycarbonsäure nicht signifikant Wechselwirkungen zu zeigen;
Q Stickstoff oder Phosphor ist; und
L ausgewählt aus der Gruppe wird, die besteht aus: 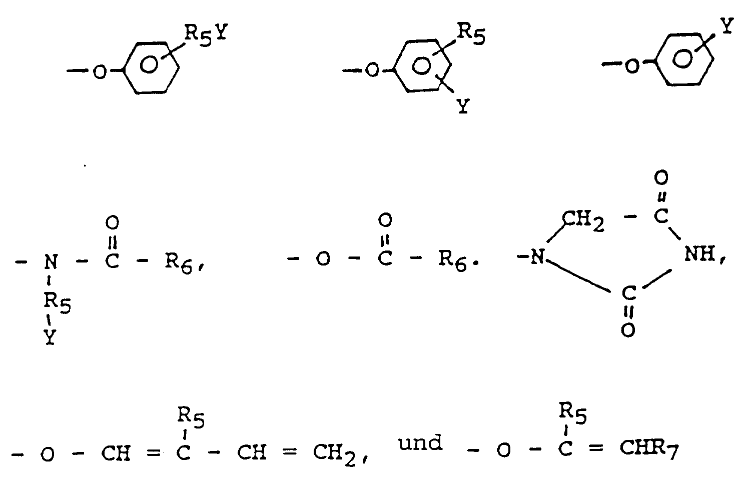 worin:
R₅ eine C₁-C₁₂ Alkyl- oder Alkylengruppe ist, R₆ eine C₁-C₁₂ Alkylgruppe ist, R₇ H oder R₅ ist, und Y H oder eine löslich machende Gruppe ist, ausgewählt aus -SO⁻₃M⁺, -COO⁻M⁺, -SO₄⁻M⁺, -N⁺(R₅)₃X⁻, -NO₂, -OH und O ← N(R₅)₂ und Mischungen davon; worin M⁺ ein Wasserstoff, ein Alkalimetall, Ammonium oder Alkyl- oder Hydroxyalkylsubstituiertes Ammoniumkation ist, X⁻ ein Halogenid, Hydroxid, Phosphat, Sulfat, Methylsulfat oder Acetatanion ist.
iii) von 0 bis 50% eines oberflächenaktiven Mittels; und
iv) von 5 bis 80% eines Waschkraftbuilders.

17. Die Zusammensetzung nach Anspruch 16, worin das oberflächenaktive Mittel im Bereich von 1 bis 40% liegt, und der Waschkraftbuilder im Bereich von 5 bis 80 Gew.% liegt.

18. Die Zusammensetzung nach Anspruch 16, worin L die Formel besitzt: 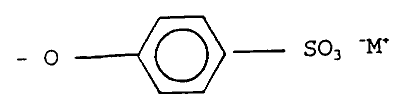 worin M⁺ ein Natrium, Kalium oder Ammoniumkation ist.

19. Die Zusammensetzung nach Anspruch 16, worin Q Stickstoff ist, und R₁, R₂ und R₃ jeweils gleich oder verschieden sind und ausgewählt werden aus C₁-C₂₀ Atomresten, ausgewählt aus der Gruppe, die besteht aus Alkyl, Alkylaryl, Benzyl, Hydroxyalkyl und heterocyclische Ringen, die den quarternären Stickstoff enthalten, wo R₁ und R₄ oder R₁ und R₂ miteinander verbunden sind, und Mischung von Gruppen davon.

20. Die Zusammensetzung nach Anspruch 19, worin R₁ ausgewählt wird aus kurzkettigen C₁-C₄ Alkylresten.

21. Die Zusammensetzung nach Anspruch 20, worin R₂ und R₃ jeweils ein langkettiger C₇-C₂₀ Alkyl- oder Alkylarylrest sind.

22. Die Zusammensetzung nach Anspruch 21, worin der längerkettige Rest ausgewählt wird aus der Gruppe, die besteht aus Benzyl, Lauryl und Stearylgruppen.

23. Die Zusammensetzung nach Anspruch 16, worin R₄ ausgewählt wird aus einer Brückengruppe, die besteht aus C₂-C₂₀ Alkylen, C₆-C₁₂ Phenylen, C₅-C₂₀ Cycloalkylen und C₈-C₂₀ Alkylenphenylengruppen.

24. Die Zusammensetzung nach Anspruch 19, worin der heterocyclische Ring aus Pyridin, Morpholin, Pyrrolidon, Piperidin und Piperazin ausgewählt wird.

25. Die Zusammensetzung nach Anspruch 16, worin Y ein Sulfonsäuresalz ist.

26. Die Zusammensetzung nach Anspruch 16, worin die Verbindung Natrium-2-(N,N,N-Trimethylammonium)ethyl-4-sulfophenylcarbonatsalz ist.

27. Die Zusammensetzung nach Anspruch 16, worin die Verbindung Natrium-2-(N-Benzyl-N,N-dimethylammonium)ethyl-4-sulfophenylcarbonatsalz ist.

28. Die Zusammensetzung nach Anspruch 16, worin die Verbindung Natrium-2-(N-Butyl-N,N-dimethylammonium)ethyl-4-sulfophenylcarbonatsalz ist.

29. Die Zusammensetzung nach Anspruch 16, worin die Verbindung Natrium-2-[4-(N,N,N-Trimethylammonium)phenyl]ethyl-4-sulfophenylcarbonatsalz ist.

30. Die Zusammensetzung nach Anspruch 16, worin die Verbindung Natrium-3-(1,1-Dimethylpiperidinium)-4-sulfophenylcarbonatsalz ist.

31. Die Zusammensetzung nach Anspruch 16, worin die Verbindung Natrium-4-(1,1-Dimethylpiperidinium)-4-sulfophenylcarbonatsalz ist.

## Revendications

1. Composé précurseur de blanchiment ayant la formule : 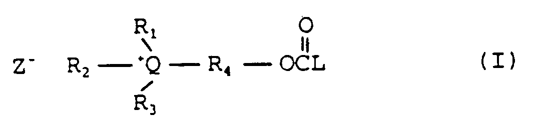 où
R₁, R₂ et R₃ sont chacun un radical choisi dans le groupe constitué par un alkyle, un alcényle, un alcynyle, un cycloalkyle, un cycloalcényle, un alkaryle, un aryle, un phényle, un hydroxyalkyle, un polyoxyalkylényle et R₄OCOL;
ou deux, ou plus, des R₁, R₂ et R₃ forment ensemble un système à noyau hétérocyclique, substitué avec un alkyle, ou non substitué, contenant de l'azote;
ou au moins un des R₁, R₂ et R₃ est fixé à R₄ de façon à former un système à noyau hétérocyclique, substitué avec un alkyle, ou non susbtitué, contenant de l'azote;
R₄ est choisi dans un groupe de pontage constitué par un alkylène, un cycloalkylène, un alkylènephénylène, un phénylène, un arylène et un polyalcoxylène, et où le groupe de pontage peut être non substitué ou substitué avec des radicaux en C₁-C₂₀ alkyle, alcényle, benzyle, phényle et aryle;
Z⁻ est un anion monovalent ou multivalent conduisant à une neutralité de charge lorsqu'il est combiné avec Q⁺ dans le rapport approprié et où Z⁻ est suffisamment stable vis-à-vis de l'oxydation pour ne pas interférer de façon significative avec le blanchiment par un acide peroxycarbonique;
Q est l'azote ou le phosphore ; et
L est choisi dans le groupe constitué par : 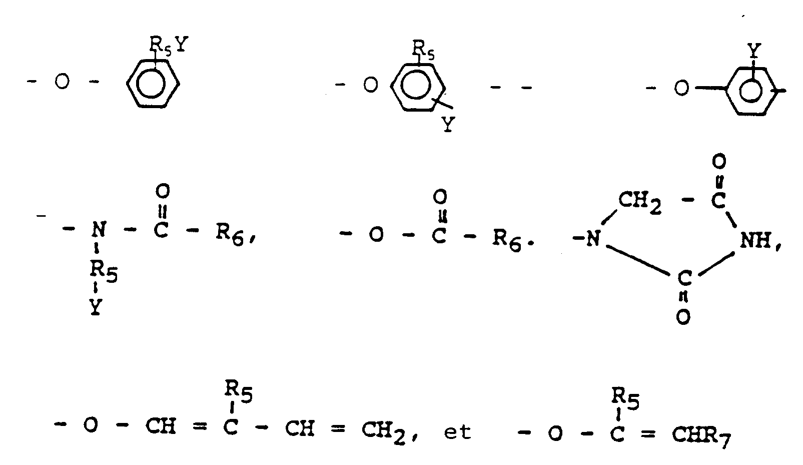 où R₅ est un groupe en C₁-C₁₂ alkyle ou alkylène, R₆ est un groupe alkyle en C₁-C₁₂, R₇ est H ou R₅, et Y est H ou un groupe de solubilisation, choisi parmi -SO⁻₃M⁺, -COO⁻M⁺, -SO⁻₄M⁺, -SO⁻₄M⁺, -N⁺(R₅)₃X⁻, -NO₂, -OH et O←N(R₅)₂ et leurs mélanges; M⁺ est un cation d'hydrogène, de métal alcalin, d'ammonium ou d'ammonium substitué par un alkyle ou un hydroxyalkyle, et X⁻ est un anion d'halogénure, d'hydroxyde, de phosphate, de sulfate, de méthylsulfate ou d'acétate.

2. Précurseur selon la revendication 1, où L a la formule : 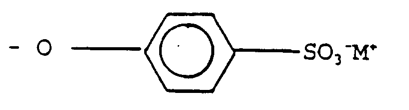 où M⁺ est un cation de sodium, de potassium ou d'ammonium.

3. Précurseur selon la revendication 1, où Q est l'azote, et R₁, R₂ et R₃ sont chacun le même ou différent et choisis parmi les radicaux en C₁-C₂₀ choisis dans le groupe constitué par un alkyle, un alkylaryle, un benzyle, un hydroxyalkyle, et les noyaux hétérocycliques contenant l'azote quaternaire où R₁ et R₄ ou R₁ et R₂ sont liés ensemble, et des mélanges de ces groupes.

4. Précurseur selon la revendication 3, où R₁ est choisi parmi les radicaux alkyles en C₁-C₄ à chaîne courte.

5. Précurseur selon la revendication 4, où R₂ et R₃ sont chacun un radical à plus longue chaîne en C₇-C₂₀ alkyle ou alkylaryle.

6. Précurseur selon la revendication 5, où ledit radical à chaîne plus longue est choisi dans le groupe constitué par les groupes benzyle, lauryle et stéaryle.

7. Précurseur selon les revendications 1 à 4, où R₄ est choisi dans un groupe de liaison constitué par les groupes alkylène en C₂-C₂₀, phénylène en C₆-C₁₂, cycloalkylène en C₅-C₂₀, et alkylènephénylène en C₈-C₂₀.

8. Précurseur selon la revendication 3, où ledit noyau hétérocyclique est choisi parmi la pyridine, la morpholine, la pyrrolidone, la pipéridine et la pipérazine.

9. Précurseur selon la revendication 1, où Y est un sel d'acide sulfonique.

10. Précurseur selon la revendication 1, où le composé est un sel de 2-(N,N,N-triméthylammonium) éthyl 4-sulfophényl carbonate.

11. Précurseur selon la revendication 1, où le composé est un sel de 2-(N-benzyl-N,N-diméthylammonium) éthyl 4-sulfophényl carbonate.

12. Précurseur selon la revendication 1, où le composé est un sel de 2-(N-butyl-N,N-diméthylammonium) éthyl 4-sulfophényl carbonate.

13. Précurseur selon la revendication 1, où le composé est un sel de 2-[4-(N,N,N-triméthylammonium) phényl] éthyl 4-sulfophényl carbonate.

14. Précurseur selon la revendication 1, où le composé est un sel de 3-(1,1-diméthylpipéridinium) 4-sulfophényl carbonate.

15. Précurseur selon la revendication 1, où le composé est un sel de 4-(1,1-diméthylpipéridinium) 4-sulfophényl carbonate.

16. Composition de blanchiment pour détergent comportant :
(i) de 1 à 60% d'un composé peroxygéné capable de donner de l'eau oxygénée dans une solution aqueuse;
(ii) de 0,1 à 40% d'un précurseur de blanchiment ayant la formule : 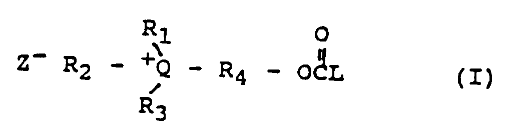
où R₁, R₂ et R₃ sont chacun un radical choisi dans le groupe constitué par un alkyle, un alcényle, un alcynyle, un cycloalkyle, un cycloalcényle, un alkaryle, un aryle, un phényle, un hydroxyalkyle, un polyoxyalkylényle et R₄OCOL;
ou deux, ou plus, des R₁, R₂ et R₃ forment ensemble un système à noyau hétérocyclique, substitué avec un alkyle, ou non substitué, contenant de l'azote;
ou au moins un des R₁, R₂ et R₃ est fixé à R₄ de façon à former un système à noyau hétérocyclique, substitué avec un alkyle, ou non substitué, contenant de l'azote;
R₄ est choisi dans un groupe de pontage constitué par un alkylène, un cycloalkylène, un alkylènephénylène, un phénylène, un arylène et un polyalcoxylène, et où le groupe de pontage peut être non substitué ou substitué avec des radicaux en C₁-C₂₀, choisi parmi les radicaux alkyle, alcényle, benzyle, phényle et aryle;
Z⁻ est un anion monovalent ou multivalent conduisant à une neutralité de charge lorsqu'il est combiné avec Q⁺ dans le rapport approprié, et où Z⁻ est suffisamment stable vis-à-vis de l'oxydation pour ne pas interférer de façon significative avec le blanchiment par un acide peroxycarbonique;
Q est l'azote ou le phosphore; et
L est choisi dans le groupe constitué par 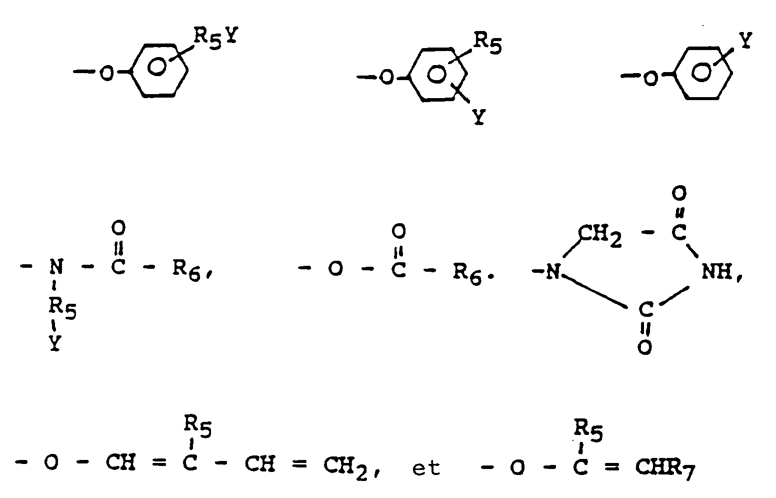 où R₅ est un groupe en C₁-C₁₂ alkyle ou alkylène, R₆ est un groupe alkyle en C₁-C₁₂, R₇ est H ou R₅, et Y est H ou un groupe de solubilisation, choisi parmi -SO⁻₃M⁺, -COO⁻M⁺, -SO⁻₄M⁺, -N⁺(R₅)₃X⁻, -NO₂, -OH, et O←N(R₅)₂ et leurs mélanges; M⁺ est un cation d'hydrogène, de métal alcalin, d'ammonium ou d'ammonium substitué par un alkyle ou un hydroxyalkyle, X⁻ est un anion d'halogénure, d'hydroxyde, de phosphate, de sulfate, de méthylsulfate ou d'acétate;
(iii) de 0 à 50% d'un agent tensio-actif; et
(iv) de 5 à 80% d'un adjuvant de détergent.

17. Composition selon la revendication 16, où l'agent tensio-actif va de 1 à 40% et l'adjuvant de détergent va de 5 à 80% en poids.

18. Composition selon la revendication 16, où L a la formule : 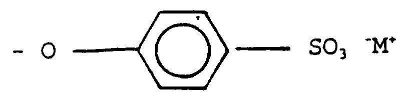 où M⁺ est un cation de sodium, de potassium ou d'ammonium.

19. Composition selon la revendication 16, où Q est l'azote et R₁, R₂ et R₃ sont chacun le même ou différent et choisis parmi les radicaux en C₁-C₂₀choisis dans le groupe constitué par les alkyles, les alkylaryles, le benzyle, les hydroxyalkyles, et les noyaux hétérocycliques contenant l'azote quaternaire où R₁ et R₄ ou R₁ et R₂ sont liés ensemble, et les mélanges de ces groupes.

20. Composition selon la revendication 19, où R₁ est choisi parmi les radicaux alkyles à courte chaîne en C₁-C₄ .

21. Composition selon la revendication 20, où R₂ et R₃ sont chacun un radical alkyle ou alkylaryle à plus longue chaîne, en C₇-C₂₀.

22. Composition selon la revendication 21, où ledit radical à plus longue chaîne est choisi dans le groupe constitué par les groupes benzyle, lauryle et stéaryle.

23. Composition selon la revendication 16, où R₄ est choisi dans un groupe de pontage constitué par les groupes alkylène en C₂-C₂₀,phénylène en C₆-C₁₂, cycloalkylène en C₅-C₂₀, et alkylènephénylène en C₈-C₂₀.

24. Composition selon la revendication 19, où ledit noyau hétérocyclique est choisi parmi la pyridine, la morpholine, la pyrrolidone, la pipéridine et la pipérazine.

25. Composition selon la revendication 16, où Y est un sel d'acide sulfonique.

26. Composition selon la revendication 16, où le précurseur est un sel de 2-(N,N,N-triméthylammonium) éthyl sodium 4-sulfophényl carbonate.

27. Composition selon la revendication 16, où le précurseur est un sel de 2-(N-benzyl-N,N-diméthylammonium) éthyl sodium 4-sulfophényl carbonate.

28. Composition selon la revendication 16, où le précurseur est un sel de 2-(N-butyl-N,N-diméthylammonium) éther sodium 4-sulfophényl carbonate.

29. Composition selon la revendication 16, où le précurseur est un sel de 2-[4-(N,N,N-triméthylammonium) phényl] éthyl sodium 4-sulfophényl carbonate.

30. Composition selon la revendication 16, où le précurseur est un sel de sodium 3-(1,1-diméthylpipéridinium) 4-sulfophényl carbonate.

31. Composition selon la revendication 16, où le précurseur est un sel de sodium 4-(1,1-diméthylpipéridinium) 4-sulfophényl carbonate.
